(19) **Europäisches Patentamt / European Patent Office / Office européen des brevets**

(11) **EP 4 142 635 B1**

(12) **FASCICULE DE BREVET EUROPÉEN**

(45) Date de publication et mention
de la délivrance du brevet:
**18.06.2025 Bulletin 2025/25**

(21) Numéro de dépôt: **21721567.2**

(22) Date de dépôt: **30.04.2021**

(51) Classification Internationale des Brevets (IPC):
*A61B 34/30* *(2016.01)*    *A61B 90/50* *(2016.01)*
*A61F 9/007* *(2006.01)*    *A61B 17/00* *(2006.01)*

(52) Classification Coopérative des Brevets (CPC):
**A61B 90/50; A61B 34/30; A61F 9/007;**
A61B 2017/0046; A61B 2017/00477;
A61B 2017/305

(86) Numéro de dépôt international:
**PCT/EP2021/061440**

(87) Numéro de publication internationale:
**WO 2021/219864 (04.11.2021 Gazette 2021/44)**

(54) **MÉCANISME DE MANIPULATION D'UN INSTRUMENT CHIRURGICAL**

MECHANISMUS ZUR HANDHABUNG EINES CHIRURGISCHEN INSTRUMENTS

MECHANISM FOR HANDLING A SURGICAL INSTRUMENT

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **30.04.2020 FR 2004320**

(43) Date de publication de la demande:
**08.03.2023 Bulletin 2023/10**

(73) Titulaires:
• **Acusurgical**
**34000 Montpellier (FR)**
• **Centre National de la Recherche Scientifique**
**75016 Paris (FR)**
• **UNIVERSITE DE MONTPELLIER**
**34090 Montpellier (FR)**

(72) Inventeurs:
• **SPUHLER, Christoph**
**34090 MONTPELLIER (FR)**
• **HADDAB, Yassine**
**34080 MONTPELLIER (FR)**
• **POIGNET, Philippe**
**34150 GIGNAC (FR)**
• **MOREL, Antoine**
**34090 MONTPELLIER (FR)**
• **SANCHEZ, Alonso**
**34990 JUVIGNAC (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
**WO-A1-2019/183236    US-A- 5 807 378
US-A1- 2018 014 849**

EP 4 142 635 B1

**Description**

**Domaine technique de l'invention**

La présente invention concerne un mécanisme d'actionnement d'un instrument chirurgical, destiné à être utilisé dans une plateforme robotisée pour la chirurgie, en particulier la chirurgie de l'œil.

**Arrière-plan technique**

Il est bien connu de la technique qu'une opération de chirurgie vitréo-rétinienne, ou communément chirurgie de l'œil, est réalisée par un praticien sur un patient par le biais d'une canule, ou trocart, placée sur la partie antérieure de l'œil et dans laquelle est inséré un instrument chirurgical. Ce trocart définit un accès qui permet notamment de traverser le vitré et de rejoindre la partie postérieure de l'œil, où se situe la rétine.

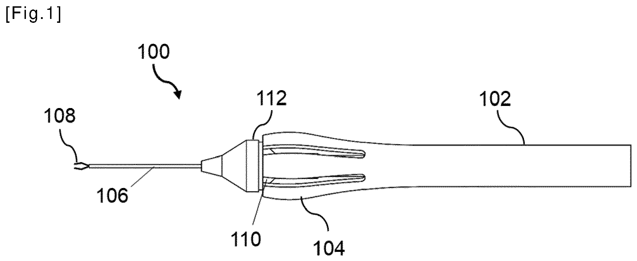

On entend par « instrument » un dispositif comme montré sur la figure 1. Classiquement, un instrument 100 typique de chirurgie de l'œil comprend un manche 102, un préhenseur 104, une gaine 106 et un outil 108.

Le manche 102 est configuré pour tenir dans une main du praticien de sorte que celui-ci puisse avoir une bonne prise. Le préhenseur 104, situé dans le prolongement du manche 102, est conçu pour que le praticien y positionne ses doigts pour manipuler l'instrument 100. En outre, le préhenseur 104 peut se déformer suite à une pression exercée par le praticien. La déformation du préhenseur 104 se traduit par le déplacement d'un élément poussoir 110, situé à l'intérieur d'une tête 112 conique, qui actionne la gaine 106. La gaine 106 glisse le long de l'outil 108 situé à son extrémité, ce qui permet de l'actionner dans le cas où l'outil 108 est destiné à une fonction de pincement, comme une pince ou une paire de ciseaux par exemple. Un organe de rappel, tel un ressort, est logé dans la tête 112 et coopère avec l'élément poussoir 110, ce qui permet à l'outil 108 actionné de reprendre sa configuration initiale lorsque la pression exercée sur le préhenseur 104 est relâchée.

L'outil est la partie fonctionnelle de l'instrument et peut prendre diverses formes. L'outil peut notamment être une pince, une paire de ciseau, un couteau, un aspirateur, un laser, une sonde cryogénique ou tout élément pouvant être employé en chirurgie.

Dans une variante, le préhenseur 104 est muni d'un système à glissière qui effectue un mouvement de translation pour actionner l'outil 108.

Dans une autre variante, l'instrument 100 est de type « backflush » et le préhenseur 104 est muni d'un bouton poussoir permettant une action de succion avec l'outil 108.

Le praticien manipule l'instrument dans l'espace mais doit faire preuve d'une dextérité particulière pour produire des mouvements avec une très faible amplitude, de seulement quelques dizaines de micromètres.

Toutefois, une intervention manuelle présente de nombreux risques pour le patient liés principalement à une erreur de manipulation du praticien.

Afin d'apporter davantage de confort, de précision et de sécurité pendant une opération chirurgicale de l'œil, une robotisation de cette chirurgie est envisagée. Pour cela, il est nécessaire que les mécanismes de manipulation et d'actionnement des instruments chirurgicaux reproduisent des mouvements similaires à ceux du praticien utilisant un instrument chirurgical.

Dans le document WO 2019/183236 A1, un instrument de chirurgie est notamment installé sur une base assurant des mouvements de rotation et de translation. Cependant, un tel appareillage présente l'inconvénient d'être encombrant, peu pratique, car nécessitant, par exemple l'installation au préalable d'un collier sur l'instrument, et ne permet pas une manipulation de tous les types d'outils pouvant être employés pour une chirurgie de l'œil. En outre, cet appareillage ne permet pas de supprimer le risque de glissement de l'instrument en direction de l'œil en cas de dissociation avec sa base.

**Résumé de l'invention**

La présente invention a pour but de résoudre l'un, au moins, des inconvénients précités. En particulier, la présente invention a pour but de proposer un mécanisme permettant de manipuler et d'actionner un instrument chirurgical de manière sûre, pratique, précise et efficace. L'invention est définie par les revendications 1 et 13.

À cet effet, l'invention propose un mécanisme d'actionnement pour un instrument chirurgical comprenant :

- un manchon, muni d'un axe X longitudinal, configuré pour recevoir l'instrument chirurgical et comportant une ou plusieurs pièces s'étendant sensiblement dans un plan perpendiculaire audit axe X longitudinal, plusieurs goujons s'étendant selon l'axe X longitudinal étant prévus sur la ou chaque pièce, sur certaines pièces seulement, ou encore répartis sur les différentes pièces ;
- au moins deux roues, montées sur le manchon de part et d'autre de la ou desdites pièces selon ledit axe X longitudinal, chaque roue étant munie d'au moins une rainure et d'un élément de transmission mécanique, ladite au moins une rainure de chaque roue recevant, de façon mobile dans ladite rainure, l'un de ladite pluralité de goujons ;

- au moins deux arbres d'entraînement, un premier arbre d'entraînement étant muni d'un premier élément de transmission mécanique coopérant avec l'élément de transmission mécanique de l'une des deux roues et un deuxième arbre d'entraînement étant muni d'un autre premier élément de transmission mécanique coopérant avec l'élément de transmission mécanique de l'autre des deux roues ;
- l'au moins une pièce est une mâchoire, montée entre des guides de sorte à pouvoir translater par rapport aux guides, les guides étant eux-mêmes montés fixes par rapport au manchon ou l'au moins une pièce est un guide, monté fixe par rapport au manchon.

Ainsi, grâce à l'invention, on assure un mouvement de l'instrument chirurgical identique à un mouvement ordinairement effectué par un praticien. En effet, le mécanisme permet de manipuler l'instrument chirurgical avec plusieurs degrés de liberté, en rotation et en translation notamment, mais permet également d'actionner toute variante de préhenseur de l'instrument. Ceci confère l'avantage de pouvoir utiliser une large gamme d'instruments chirurgicaux standards, disponibles sur étagère, et sans nécessiter une modification matérielle de ces derniers.

En outre, grâce à l'invention, on assure une sécurisation du patient accrue. En effet, le mécanisme permet une grande précision de déplacement de l'instrument chirurgical qui est maintenu vers l'avant par le mécanisme, empêchant ainsi tout glissement accidentel de l'instrument vers le patient et en particulier vers l'œil. Encore, grâce à l'invention, on assure une amélioration de l'intégration des mécanismes de manipulation et donc un faible encombrement de l'espace de travail, ce qui permet au praticien de garder le patient dans son champ de vision et, en outre, permet l'utilisation d'autres appareillages à proximité comme par exemple un microscope ou un autre appareillage semblable à l'invention.

Le mécanisme d'actionnement selon l'invention peut comprendre une ou plusieurs des caractéristiques suivantes, prises isolément les unes des autres ou en combinaison les unes avec les autres :

- la rainure est ménagée sur une première face et l'élément de transmission mécanique est situé sur une deuxième face de chaque roue ;
- la rainure et l'élément de transmission mécanique sont situés sur une même face de la roue ;
- le manchon comporte une butée interne s'étendant radialement au niveau d'une première extrémité dudit manchon, et des languettes flexibles s'étendant axialement au niveau d'une seconde extrémité ;
- l'élément de transmission mécanique est un engrenage ou une poulie entraînée par courroie ;
- la rainure présente une distance radiale par rapport au centre de la roue qui varie le long de la rainure ;
- la rainure présente une distance radiale par rapport au centre de la roue, laquelle est constante le long de la rainure ;
- la rainure présente une profondeur variable le long de la rainure
- chaque roue comprend au moins une première rainure présentant une distance radiale par rapport au centre de la roue qui varie le long de la première rainure et présentant une profondeur variable le long de la première rainure, et au moins une seconde rainure, différente de la première rainure présentant une distance radiale par rapport au centre de la roue qui est constante le long de la seconde rainure et présentant une profondeur variable le long de la seconde rainure.

La présente invention concerne également un module caractérisé en ce qu'il comprend un mécanisme d'actionnement tel que décrit dans ce qui précède, le mécanisme étant logé dans un compartiment de protection, maintenant en interaction les éléments du mécanisme d'actionnement.

Le module selon l'invention peut comprendre une ou plusieurs des caractéristiques suivantes, prises isolément les unes des autres ou en combinaison les unes avec les autres :

- il comprend un réceptacle amovible configuré pour recevoir le compartiment de protection, le réceptacle étant fixé à une extrémité d'un appareillage ;
- au moins deux arbres parallèles d'entraînement, reliés mécaniquement à un élément de motorisation, se trouvent en saillie de l'extrémité de l'appareillage, chaque arbre étant configuré pour coopérer avec un second élément de transmission mécanique d'un des au moins deux arbres d'entraînement du mécanisme d'actionnement ;

- une housse de protection est intégrée au réceptacle de sorte que la housse entoure une partie opposée au module ;
- le réceptacle, le compartiment, le mécanisme et la housse sont des équipements stériles à usage unique.

La présente invention concerne également un procédé de mise en œuvre d'un mécanisme d'actionnement tel que décrit précédemment, le procédé étant réalisé en dehors du patient, dans lequel une rotation dans le même sens des roues engendre une rotation du manchon autour de son axe longitudinal.

La présente invention concerne également un procédé de mise en œuvre d'un mécanisme d'actionnement tel que décrit précédemment, le procédé étant réalisé en dehors du patient, dans lequel une rotation dans des sens opposés des roues engendre une translation radiale d'au moins une pièce de sorte que la pièce exerce une pression sur l'instrument chirurgical.

La présente invention concerne également un procédé de mise en œuvre d'un mécanisme d'actionnement tel que décrit précédemment, le procédé étant réalisé en dehors du patient, dans lequel une rotation dans des sens opposés des roues engendre une translation longitudinale d'au moins une pièce le long de l'instrument chirurgical.

La présente invention concerne également un procédé de mise en œuvre d'un mécanisme d'actionnement tel que décrit précédemment, le procédé étant réalisé en dehors du patient, dans lequel une rotation des roues dans des sens opposés engendre simultanément une translation longitudinale et une translation radiale d'au moins une pièce de sorte que ladite pièce exerce une pression sur l'instrument chirurgical tout en se déplaçant longitudinalement le long de l'instrument chirurgical.

La présente invention concerne également un procédé de mise en œuvre d'un mécanisme d'actionnement tel que décrit précédemment, le procédé étant réalisé en dehors du patient, dans lequel une rotation des roues dans des sens opposés engendre simultanément une translation longitudinale d'au moins une pièce le long de l'instrument chirurgical et une translation radiale d'au moins une autre pièce de sorte que l'autre pièce exerce une pression sur l'instrument chirurgical.

## Brève description des figures

D'autres caractéristiques et avantages de l'invention apparaîtront au cours de la lecture de la description détaillée qui va suivre pour la compréhension de laquelle on se reportera aux dessins annexés dans lesquels :

[Fig. 1] la figure 1 est une vue schématique de profil d'un instrument chirurgical standard ;

[Fig. 2] la figure 2 est une vue schématique en perspective d'un module portant un mécanisme d'actionnement de l'instrument chirurgical de la figure 1, selon une forme de réalisation de l'invention ;

[Fig. 3] la figure 3 est une vue schématique éclatée de la figure 2 ; [Fig. 4] la figure 4 est une vue schématique en coupe d'un mécanisme d'actionnement logé dans un compartiment de protection ;

[Fig. 5] la figure 5 est une vue schématique éclatée en perspective de la figure 4 ;

[Fig. 6A] la figure 6A est une vue schématique du mécanisme à came différentielle dans une position ouverte des mâchoires selon un mode de réalisation de l'invention ;

[Fig. 6B] la figure 6B est une vue schématique du mécanisme de la figure 6A, après rotation simultanée d'un quart de tour des roues mobiles dans des sens opposés ;

[Fig. 6C] la figure 6C est une vue schématique du mécanisme de la figure 6A, après rotation simultanée d'un quart de tour des roues mobiles dans le même sens ;

[Fig. 7A] la figure 7A est une vue schématique du mécanisme à came différentielle dans une position initiale selon un autre mode de réalisation de l'invention ;

[Fig. 7B] la figure 7B est une vue schématique selon un axe de coupe de la figure 7A ;

[Fig. 7C] la figure 7C est une vue schématique selon l'axe de coupe de la figure 7A, après rotation simultanée d'un quart de tour des roues mobiles dans des sens opposés ;

[Fig. 8] la figure 8 est une vue schématique éclatée en perspective d'un mécanisme d'actionnement selon une autre forme de réalisation ; et

[Fig. 9] la figure 9 est une vue schématique éclatée en perspective d'un mécanisme d'actionnement selon une autre forme de réalisation.

## Description détaillée de l'invention

Dans la description détaillée qui suit, il est fait référence à un dispositif destiné à être employé durant une intervention chirurgicale. Il est à noter que les exemples de réalisation décrits sont tous réalisés en dehors de toute opération de chirurgie et par conséquent en dehors du patient.

Dans ce qui suit, on se réfère à un instrument chirurgical standard similaire à l'instrument décrit dans ce qui précède à la figure 1.

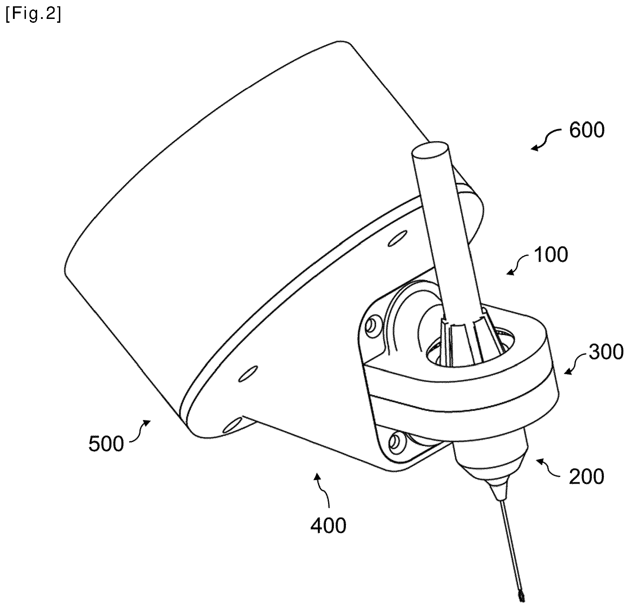

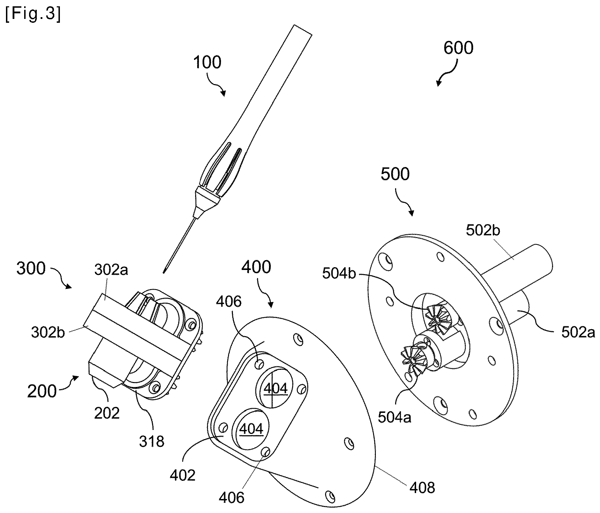

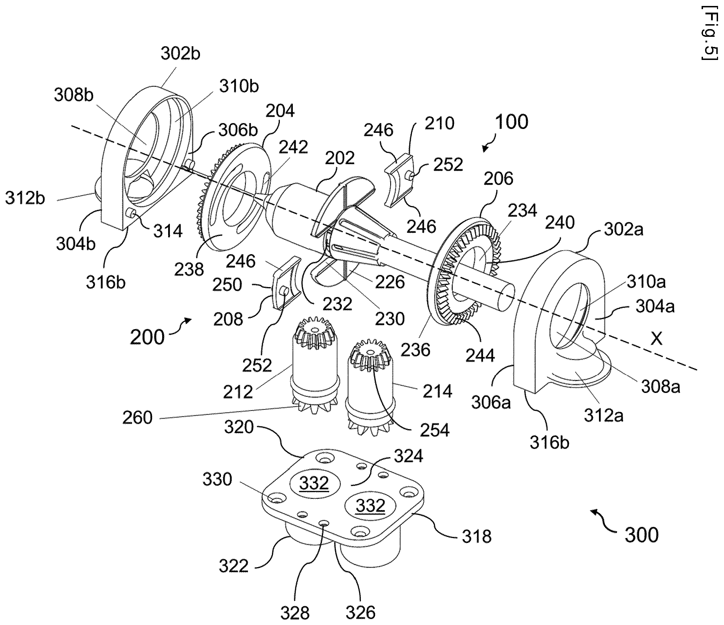

On se réfère à présent aux figures 2 à 5, illustrant un mécanisme d'actionnement d'un instrument chirurgical selon une forme de réalisation.

Les opérations chirurgicales touchant l'œil peuvent être réalisées à l'aide d'un appareillage ou d'une plateforme robotisée. L'appareillage comprend des éléments de motorisation qui lui permettent de se mouvoir avec plusieurs degrés de liberté. Afin de manipuler et actionner l'instrument 100 chirurgical comme le ferait un praticien, l'appareillage peut être équipé d'un module 600 muni d'un mécanisme 200 d'actionnement de l'instrument 100 pouvant être compris dans un compartiment 300 de protection fixé sur un réceptacle 400 situé à l'extrémité 500 de l'appareillage.

Le mécanisme 200 d'actionnement comprend par ailleurs un manchon 202, au moins deux roues 204, 206 mobiles, au moins une pièce 208, 210, que l'on nommera mâchoire, et au moins deux arbres 212, 214 d'entraînement.

Le manchon 202 comporte un corps 216 principal de forme globalement cylindrique, s'étendant le long d'un axe X longitudinal. Le corps 216 est évidé selon cet axe X et ouvert à une extrémité 218 amont et à une extrémité 220 aval de façon à recevoir l'instrument 100.

L'extrémité 218 amont du manchon 202 comporte des languettes 222 s'étendant axialement et réparties radialement autour de l'axe X longitudinal. Les languettes 222 sont flexibles de façon à pouvoir se déformer et s'écarter les unes des autres au passage de l'instrument 100 dans le manchon 202.

L'extrémité 220 aval du manchon 202 forme un rétrécissement annulaire et présente une butée 224 interne s'étendant radialement. Cette butée 224 permet de retenir vers l'avant l'instrument 100 chirurgical lorsqu'il est inséré dans le manchon 202.

On comprend que l'instrument 100 chirurgical peut être inséré dans le manchon 202 par l'extrémité 218 amont. En outre, le diamètre de l'extrémité 220 aval du manchon est sensiblement inférieur au diamètre de la base de la tête 112 de l'instrument 100 de sorte que l'instrument 100 ne se désengage pas du mécanisme 200 pendant son utilisation. Ceci présente l'avantage de garantir la sécurité du patient. En effet, l'instrument 100 chirurgical est maintenu vers l'avant par la butée 224 interne, empêchant ainsi tout glissement accidentel de l'instrument 100 vers le patient et en particulier vers son œil.

Le manchon 202 comporte également au moins un guide 226, monté fixe par rapport au manchon 202, par exemple venu de matière avec le manchon 202, s'étendant sensiblement dans un plan P, perpendiculaire à l'axe X longitudinal. Dans la forme de réalisation présentée ici, le manchon 202 comporte deux guides 226, distribués de manière équidistante autour du manchon 202. Chaque guide 226 est muni de deux pistes 230, planes, s'étendant de part et d'autre du manchon 202 selon un axe perpendiculaire à l'axe X longitudinal. Chaque piste 230 d'un guide 226 est située face à la piste 230 de l'autre des deux guides 226 de sorte que les pistes 230 sont parallèles.

Le manchon 202 comprend encore au moins une ouverture 232 périphérique qui est située sur la périphérie du corps 216 principal. Dans la forme de réalisation présentée ici, le manchon 202 comporte deux ouvertures 232. Ces ouvertures 232, distribuées de manière équidistante autour du manchon, s'étendent circonférentiellement entre les guides 226 et se trouvent dans le même plan P perpendiculaire que les guides 226. La largeur des ouvertures 232 peut être sensiblement égale ou sensiblement supérieure à la largeur des pistes 230 des guides 226.

Les deux roues 204, 206 mobiles de forme annulaire sont montées axialement sur le manchon 202 de part et d'autre des guides 226. On comprend que les guides 226 servent aussi d'entretoises entre les roues 204, 206. Chaque roue 204, 206 présente un axe longitudinal se confondant avec l'axe X longitudinal du manchon 202. Chaque roue 204, 206 comporte par ailleurs un bord 234 périphérique interne et un bord 236 périphérique externe, concentriques et centrés sur l'axe X longitudinal, une première face 238 et une seconde face 240, opposée à la première, et orientées dans la direction de l'axe X longitudinal. La première face 238 comprend au moins une rainure 242, creusée dans une portion non nulle de l'épaisseur de la roue 204, 206, et fait face aux guides 226. La rainure 242 comprend deux extrémités et décrit une trajectoire. Cette trajectoire peut par exemple, de manière non limitative, être un arc, concentrique ou non avec la roue 204, 206.

La seconde face 240 est munie d'un élément 244 de transmission mécanique. Dans la forme de réalisation présentée ici, l'élément 244 est un engrenage mais peut aussi être une poulie configurée pour être entraînée par courroie.

Dans une variante, non représentée ici, la rainure 242 et l'élément 244 de transmission mécanique peuvent se situer sur une même face 238 de la roue 204, 206. On comprend que la rainure 242 et l'élément 244 de transmission mécanique sont situés sur la même face 238, celle-ci étant située en regard de l'au moins un guide 226.

Dans une autre variante, non représentée, l'élément 244 de transmission mécanique peut-être situé sur le bord 236 périphérique externe de la roue 204, 206.

L'au moins une mâchoire 208, 210, s'étend sensiblement dans le plan P perpendiculaire à l'axe X longitudinal. La mâchoire 208, 210 est montée sur le manchon 202 de sorte à pouvoir translater par rapport à l'au moins un guide 226. La mâchoire 208, 210 comporte par ailleurs deux pistes 246 opposées, chacune située sur une tranche de la mâchoire 208, 210 et dont la largeur est sensiblement égale à l'épaisseur de la mâchoire 208, 210. Les pistes 246 de la mâchoire 208, 210 coopèrent avec les pistes 230 des guides 226. Autrement dit, les pistes 246 des mâchoires 208, 210 glissent sur les pistes 230 des guides 226.

On comprend que la largeur des pistes 246 d'une mâchoire est sensiblement inférieure ou égale à la largeur d'une piste 230 d'un guide 226. La largeur des pistes 246 est en outre sensiblement inférieure à la largeur d'une ouverture 232. Autrement dit, la largeur de l'ouverture 232 est sensiblement supérieure à l'épaisseur de la mâchoire 208, 210.

Dans la forme de réalisation présentée ici, le mécanisme 200 comprend deux mâchoires 208, 210, réparties de manière équidistante entre les guides 226.

Une première mâchoire 208 comporte sur au moins l'une de ses faces, un premier goujon 250, orienté axialement, logé dans la au moins une rainure 242 d'une première roue 204.

Une deuxième mâchoire 210, similaire à la première mâchoire 208, comporte sur au moins l'une de ses faces, un deuxième goujon 252, orienté axialement, logé dans la au moins une rainure 242 de l'autre roue 206.

La première mâchoire 208 peut comporter sur son autre face un autre goujon 252, orienté axialement dans le prolongement du premier goujon 250, logé dans une deuxième rainure 242 de l'autre roue 206.

La deuxième mâchoire 210 peut comporter sur son autre face, un autre goujon 250, orienté axialement dans le prolongement du deuxième goujon 252, logé dans une deuxième rainure 242 de la première roue 204.

On comprend que chaque mâchoire 208, 210 peut comprendre un ou plusieurs goujons 250, 252, s'étendant parallèlement à l'axe X longitudinal du manchon 202. Dans un premier cas, chaque mâchoire 208, 210 comporte un goujon 250 sur une première face ou un goujon 252 sur une seconde face. Si la première mâchoire 208 comporte un goujon 250 sur sa première face alors la deuxième mâchoire 210 comporte un goujon 252 sur sa seconde face, et inversement. Dans un deuxième cas, chaque mâchoire 208, 210 comporte un goujon 250 sur sa première face et un goujon 252 sur sa seconde face, en d'autres termes, un goujon 250, 252 par face. En outre, chaque mâchoire 208, 210 peut coopérer avec une des deux roues 204, 206 ou avec les deux roues 204, 206 par l'intermédiaire du ou des goujons 250, 252, chacun coopérant avec une rainure 242.

La largeur de la rainure 242 est sensiblement égale ou supérieure à la section du goujon 250, 252 de la mâchoire 208, 210. Le goujon 250, 252 coopère avec la rainure 242 de façon à suivre le tracé formé par la rainure 242 lors de la rotation de la roue 204, 206. Le goujon 250, 252 est donc mobile dans la rainure 242. En d'autres termes, un mécanisme de came à rainure est utilisé et en particulier une transmission à came différentielle qui contribue au déplacement de la mâchoire 208, 210.

On comprend donc que le mécanisme 200 d'actionnement comporte une ou plusieurs mâchoires 208, 210 montée(s) mobile(s) par rapport au manchon 202 de sorte à pouvoir translater par rapport à au moins un guide 226. La ou chaque mâchoire 208, 210 comporte, en outre, un ou plusieurs goujons 250, 252 de sorte que l'au moins une rainure 242 de chaque roue 204, 206 reçoive, de façon mobile, un goujon 250, 252.

Le mécanisme 200 comprend aussi au moins deux arbres 212, 214 d'entraînement, chacun reliés mécaniquement à un élément de motorisation. Le premier arbre 212 d'entraînement est muni d'un premier élément 254 de transmission mécanique coopérant avec l'élément 244 de transmission mécanique de la roue 204 et le deuxième arbre d'entraînement 214 est muni d'un autre premier élément 254 de transmission mécanique coopérant avec l'élément 244 de transmission mécanique de l'autre roue 206. Dans l'exemple représenté ici, chacun des éléments 254 de transmission mécanique des arbres 212, 214 d'entraînement est un engrenage coopérant avec les engrenages 244 des roues 204, 206.

Chaque arbre 212, 214 est logé dans une gaine 256 de forme globalement cylindrique dans laquelle l'arbre 212, 214 peut se mouvoir en rotation. Le premier élément 254 de transmission mécanique est situé à une extrémité de la gaine 256. Un second élément 260 de transmission mécanique est situé à l'autre extrémité de la gaine 256. Dans le présent exemple, les éléments 254 et 260 sont des engrenages.

Dans une variante, non représentée ici, les éléments 254 et 260 peuvent être des poulies. L'élément 254 peut être une poulie entraînant une courroie pour actionner l'élément 244 de transmission mécanique d'une roue 204, 206. Dans un tel cas, l'élément 244 de transmission mécanique d'une roue 204, 206 est une poulie entraînée par la courroie. L'élément 260 de transmission mécanique peut être également une poulie actionnée par une courroie.

Dans une autre variante, les éléments 244, 254, 260 de transmission mécanique peuvent être des engrenages et/ou des poulies actionnées par courroie.

Dans une autre forme de réalisation, non représentée ici, le mécanisme 200 est identique à ce qui a déjà été décrit dans ce qui précède, excepté que le mécanisme 200 est muni d'une unique mâchoire 208, 210. Dans cette forme de réalisation, la mâchoire 208, 210 comprend un goujon 250, 252 sur chacune de ses faces, de sorte que le premier goujon 250 soit monté mobile dans la au moins une rainure 242 de la première roue 204 et que le second goujon 252 soit monté mobile dans la au moins une rainure 242 de l'autre roue 206.

Le mécanisme 200 précédemment décrit peut être logé dans un compartiment 300. Ce compartiment 300 permet en outre de maintenir en place les différents éléments du mécanisme 200 d'actionnement pour que ces éléments interagissent entre eux et contribue à la compacité du dispositif.

Le compartiment 300 est composé d'une coque supérieure 302 et d'une coque inférieure 318 conçues pour coopérer entre elles.

La coque supérieure 302 est en outre constituée d'une partie 302a amont et d'une partie 302b aval. Chaque partie 302a, 302b est monobloc et présente une forme globalement rectangulaire comportant une face 304a, 304b externe et une face 306a, 306b interne dans laquelle est ménagée une ouverture 308a, 308b circulaire. Chaque partie 302a, 302b comporte également dans l'axe de l'ouverture 308a, 308b un évidemment 310a, 310b cylindrique sur la face 304a, 304b interne, conçu pour recevoir une des roues 204, 206. Chaque partie 302a, 302b comprend à sa base une excroissance 312a, 312b évidée semi-circulaire s'étendant dans un plan perpendiculaire à l'ouverture 308a, 308b et conçue pour recouvrir le premier engrenage 254 d'un arbre 212, 214 d'entraînement. La portion évidée de l'excroissance 312a, 312b communique avec l'évidemment 310a, 310b de sorte que le premier engrenage 254 coopère avec l'engrenage 244 de la roue 204, 206. On comprend donc que le diamètre de l'évidemment 310a, 310b cylindrique est sensiblement supérieur ou égal au diamètre des roues 204, 206. On comprend également que les parties amont 302a et aval 302b sont respectivement montées autour de l'extrémité 218 amont et de l'extrémité 220 aval du manchon 202. En d'autres termes, le diamètre de l'ouverture 308a de la partie 302a amont est sensiblement égal ou supérieur au diamètre du corps 216 du manchon 202 et le diamètre de l'ouverture 308b de partie 302b aval est sensiblement égal ou supérieur au diamètre de l'extrémité 220 aval.

La partie 302b aval comporte en outre sur sa face 306b interne au moins deux tétons 314 et sur sa tranche 316b inférieure au moins deux autres tétons, non visibles sur les figures. La partie 302a amont comporte en outre sur sa face 306a interne, bien que non visibles sur les figures, au moins deux trous borgnes et sur sa tranche 316a inférieure au moins deux tétons. Les tétons 314 de la partie 302b aval sont configurés pour coopérer avec des trous borgnes situés sur la face 306a interne de la partie 302a amont.

La coque 318 inférieure est un ensemble monobloc comportant une base 320 et au moins deux protubérances 322. La base 320, sensiblement plane et rectangulaire, comprend en outre une face 324 supérieure et une face 326 inférieure. Au moins quatre trous 328 borgnes sont ménagés deux à deux sur la face 324 supérieure. Au moins quatre tenons 330, sont équitablement répartis sur les bords de la face 326 inférieure de la base 320.

Les protubérances 322 cylindriques sont situées en saillies de la face 326 inférieure. Chacune de ces protubérances 322 est évidée et ouverte à ses deux extrémités de sorte qu'une extrémité débouche sur la face 324 supérieure de la base 320 par un orifice 332. Les protubérances 322 sont configurées pour recevoir les arbres 212, 214 d'entraînement.

La coque 302 supérieure est montée sur la coque 318 inférieure de sorte que les tétons de chacune des parties 302a, 302b amont et aval s'engagent dans les trous 328 borgnes de la base.

De la sorte, le mécanisme 200 d'actionnement est protégé et maintenu en place par le compartiment 300 de protection. Les différents éléments peuvent ainsi se mouvoir à l'intérieur du compartiment 300, ce qui facilite leur intégration.

Un réceptacle 400 configuré pour recevoir le compartiment 300 précédemment décrit peut être fixé à l'extrémité 500 de l'appareillage. Le réceptacle 400 est en outre amovible, c'est-à-dire qu'il peut être retiré de l'extrémité 500.

Le réceptacle 400 comprend une embase 402 correspondant à l'empreinte de la coque 318 inférieure du compartiment 300 pour pouvoir recevoir le compartiment 300 par clipsage.

On comprend que cette embase 402 comporte au moins deux orifices 404 conçus pour le passage des protubérances 322 cylindriques et au moins quatre mortaises 406 conçues pour coopérer avec les tenons 330 de la coque 318 inférieure. La section des mortaises 406 est sensiblement égale ou inférieure à la section des tenons 330 afin que ces derniers entrent en force dans les mortaises 406. De la sorte, le compartiment 300 est solidaire du réceptacle 400. On comprend également que le compartiment 300 est amovible et peut être retiré du réceptacle 400.

Le réceptacle 400 peut par ailleurs comporter à sa base 408 une barrière de protection, sous forme de housse, conçue pour recouvrir et confiner une partie de l'appareillage, en particulier la partie ne portant pas le module 600, de sorte à l'isoler du champ stérile autour du patient. Cette housse peut être stérile.

Dans une variante, non représentée ici, le compartiment 300 possède un moyen de fixation par vissage. Dans un tel cas, des trous débouchant filetés se substituent aux tenons 330 et aux mortaises 406. Un tel moyen de fixation rend toutefois plus long le temps nécessaire pour inter-changer des outils que la solution par clipsage décrite dans ce qui précède.

En fonctionnement, un instrument 100 chirurgical standard, disponible sur étagère peut être inséré dans le manchon 202 du mécanisme 200 d'actionnement. L'instrument 100 peut notamment être retiré manuellement par le praticien et être remplacé par un autre instrument, différent du premier, au cours de l'opération chirurgicale.

Un élément de motorisation entraîne au moins deux arbres 502a, 502b, d'entraînement logés à l'intérieur de l'appareillage. Les extrémités de ces arbres 502a, 502b, non entraînées par l'élément de motorisation, sont en saillies de l'extrémité 500 et sont chacune munies d'un élément de transmission mécanique 504a, 504b. Ces éléments 504a, 504b sont configurés pour coopérer avec les éléments 260 des arbres 212, 214 d'entraînement du mécanisme 200 d'actionnement et leur transmettre un mouvement de rotation. Les arbres 212, 214 d'entraînement coopèrent avec les éléments 244 de transmission mécanique des roues 204, 206 et les entraînent en rotation autour de l'axe X longitudinal.

L'élément de motorisation peut entraîner la rotation des arbres 502a, 502b dans les deux sens et chacun des arbres 502a, 502b peut tourner dans un même sens ou dans un sens opposé. Par voie de conséquence, la rotation des roues 204, 206 peut donc se faire dans un même sens ou dans un sens opposé.

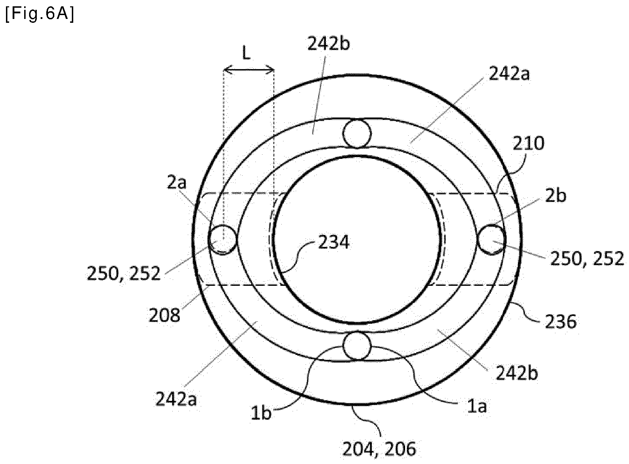

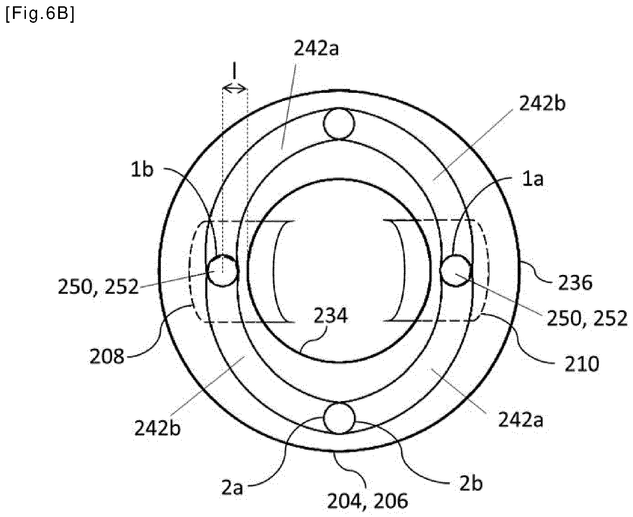

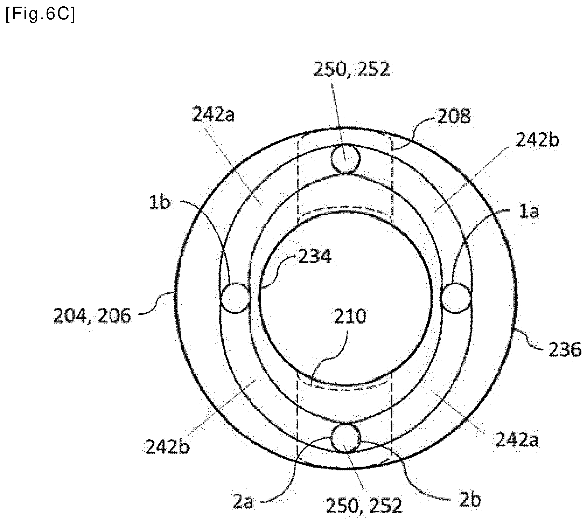

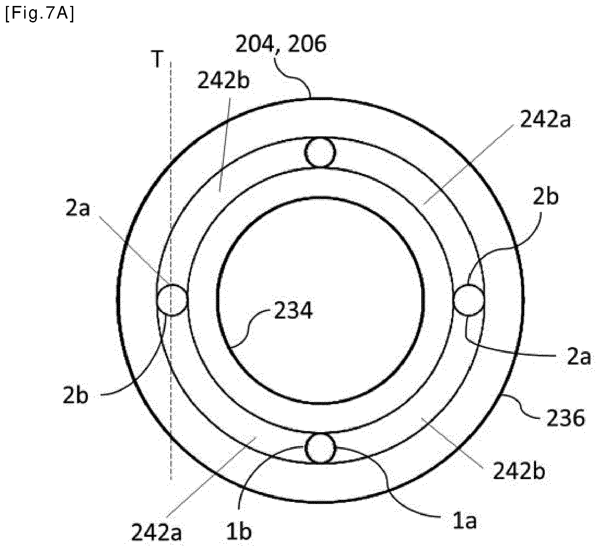

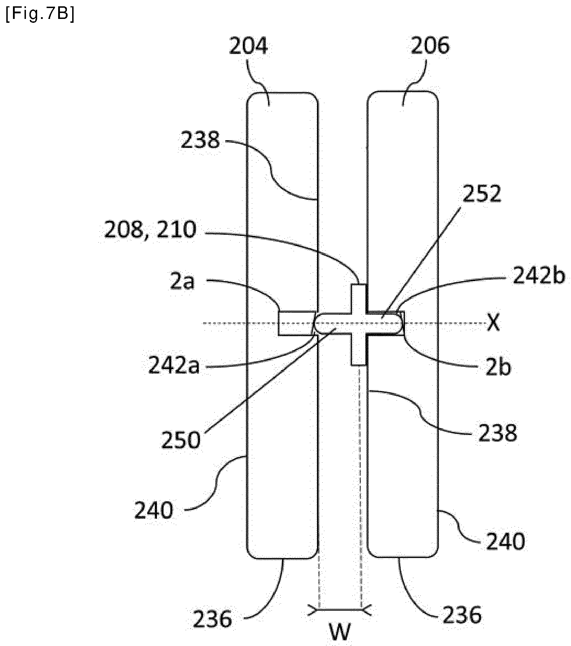

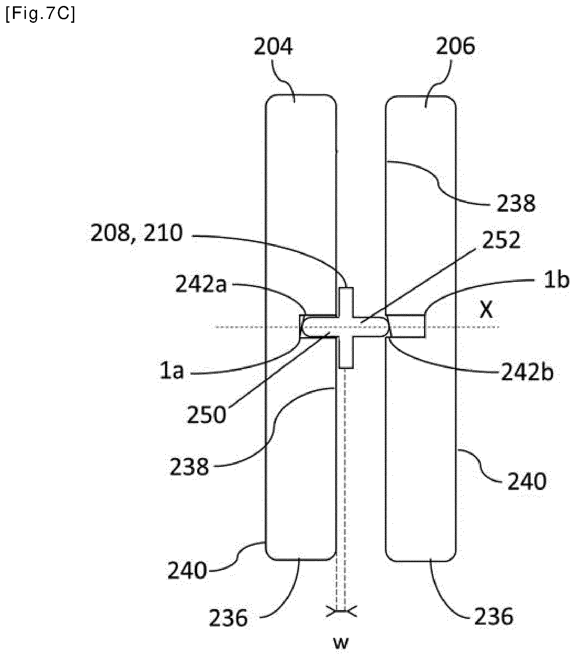

On se réfère à présent aux figures 6 et 7 illustrant les dessins de came générant des mouvements désirés pour l'actionnement de l'instrument 100 chirurgical.

Dans les exemples de réalisation présentés ici, chaque roue 204, 206 comporte deux rainures 242, ou cames 242, identiques diamétralement opposées, mais peut également en comporter une seule ou en comporter plus. Ainsi, la roue 204 comprend les rainures 242a et la roue 206, les rainures 242b. En fonctionnement, la première roue 204 et la seconde roue 206 coopèrent avec au moins une mâchoire 208, 210 de sorte que les goujons 250, 252 de l'au moins une mâchoire 208, 210 sont à la fois engagés dans la came 242a de la première roue 204 et dans la came 242b de la seconde roue 206. La position des goujons 250, 252 de la mâchoire 208, 210 correspond sensiblement à l'intersection d'une came 242a de la première roue 204 avec la projection d'une came 242b de la seconde roue 206 transposée sur le même plan que la première roue 204.

La rotation simultanée des roues 204, 206 mobiles entraîne l'actionnement de la came 242 et génère un mouvement des mâchoires 208, 210. Ce mouvement est prédéterminé par la forme du tracé de la came 242 et le sens de rotation des roues 204, 206.

<u>Première option : rotation ou pincement</u>

Sur les figures 6A à 6C, la came 242a, 242b décrit un arc non concentrique avec la roue 204, 206. Une première extrémité 1a, 1b de la came 242a, 242b est située à proximité du bord 234 périphérique interne de la roue 204, 206 tandis qu'une seconde extrémité 2a, 2b de la came est située à proximité du bord 236 périphérique externe de la roue 204, 206. Autrement dit, la distance séparant la rainure 242a, 242b du centre de la roue 204, 206 varie le long de la rainure 242a, 242b.

Dans cette configuration, la came 242a, 242b suit une trajectoire en spirale, définie par l'équation :

[Math.1]

$$r = a_0 + b\phi, b = \frac{a_1 - a_0}{2\pi n}, n \in \mathbb{R}[0, 1]$$

Où r représente la position de la mâchoire 208, 210 dans la spirale à l'intérieur de l'intervalle $[a_0, a_1]$ appartenant à un nombre réel, $\phi$ représente l'angle et $n$ représente le nombre de tours de la spirale.

La rotation simultanée de chaque roue 204, 206 dans un sens donné donne lieu à une transmission différentielle dont le fonctionnement est donné par les équations suivantes :

[Math.2]

$$\rho = \frac{\theta_1 + \theta_2}{2}, \rho = n\eta, \eta \in \mathbb{R}[0, 1]$$

[Math.3]

$$\theta = (\theta_1 - \theta_2)\pi$$

Où $\theta_1$ et $\theta_2$ représentent les positions angulaires des roues 204, 206 mobiles, $\theta$ est la position angulaire de la mâchoire 208, 210, $\rho$ est la position radiale de la mâchoire, $\eta$ est le pourcentage de serrage de la mâchoire 208, 2010 dans la plage $[a_0, a_1]$ permis par l'intersection des spirales des cames 242a, 242b.

Cela signifie que dans le système de transmission à came différentielle : la somme pondérée des rotations des roues 204, 206 ($\theta_1 + \theta_2$), engendre un premier mouvement de la mâchoire 208, 210 relatif à la forme prédéterminée de la came 242 et que la différence pondérée des roues 204, 206 ($\theta_1 - \theta_2$), engendre un deuxième mouvement de rotation de la mâchoire 208, 210 autour de l'axe de rotation des roues 204, 206.

En d'autres termes, un déplacement sélectif de la mâchoire 208, 210 est rendu possible. En effet, une rotation des roues 204, 206 autour de l'axe X longitudinal dans des sens opposés engendre une translation radiale de la mâchoire 208, 210, sans engendrer un déplacement de la mâchoire 208, 210 autour de l'axe X. Une rotation des roues 204, 206 dans le même sens engendre un déplacement de la mâchoire 208, 210 autour de l'axe X longitudinal. La mâchoire 208, 210 étant située entre les guides 226, montés fixes par rapport au manchon 202, par exemple venus de matière avec le manchon 202, son déplacement contraint le manchon 202 à suivre un mouvement identique par voie d'entraînement. Autrement dit, une rotation des roues 204, 206 dans le même sens engendre une rotation du manchon 202 et donc de l'instrument 100 chirurgical, ce qui permet de réorienter l'outil 108 manipulé. Par ailleurs, la rotation des roues 204, 206 dans le même sens n'engendre pas de déplacement radial de la mâchoire 208, 210.

Sur la figure 6A, les mâchoires 208, 210 sont dans une position dite ouverte où le goujon 250, 252 de chaque mâchoire 208, 210 se trouve à une distance L du bord 234 périphérique interne des roues 204, 206 de sorte que la mâchoire 208, 210 est comprise entre le bord 236 périphérique externe et le bord périphérique 234 interne.

Une rotation d'un quart de tour peut être effectuée pour chaque roue 204, 206, dans des sens opposés comme dans la figure 6B ou dans un même sens comme dans la figure 6C.

Avec une rotation simultanée d'un quart de tour dans des sens opposés, les mâchoires 208, 210 sont dans une position dite fermée, comme illustrée sur la figure 6B, où le goujon 250, 252 de chaque mâchoire se trouve à une distance I du bord 234 périphérique interne. Dans cette position, les mâchoires 208, 210 dépassent du bord et réduisent le diamètre de l'espace circulaire. Les mâchoires 208, 210 se déplacent donc radialement sur une distance correspondant à la différence entre la distance L et la distance I. Les mâchoires 208, 210 glissent sur les pistes 230 des guides 226 et s'insèrent dans les ouvertures 232 périphériques du manchon 202. La translation radiale des mâchoires 208, 210 permet d'exercer une pression sur l'instrument 100 chirurgical serti dans le manchon 202. En particulier, à la fin de la translation, la mâchoire 208, 210 appuie sur le préhenseur 104 de l'instrument, ce qui permet d'actionner l'outil 108 chirurgical comme décrit à la figure 1 dans ce qui précède. Ce mode de réalisation s'avère donc très utile lorsque l'outil 108 chirurgical est une pince ou une paire de ciseaux.

Avec une rotation simultanée d'un quart de tour dans le même sens, les mâchoires 208, 210 restent dans leur position initiale, soit ouverte ou fermée, et ne se déplacent pas radialement. En revanche, les mâchoires 208, 210 sont entraînées en rotation autour de l'axe X comme illustré sur la figure 6C. On comprend que la rotation du manchon 202 peut se faire avant ou après l'actionnement de l'outil 108 chirurgical.

Il est à noter que la forme de la came présentée ici n'est pas limitative. En effet, la came peut adopter toute forme conduisant à une trajectoire permettant la translation radiale ou la rotation d'au moins une mâchoire.

Par exemple, la came peut prendre la forme d'une ligne droite dont une première extrémité de la ligne est située à proximité du bord 234 périphérique interne de la roue 204, 206 tandis qu'une seconde extrémité de la ligne est située à proximité du bord 236 périphérique externe de la roue 204, 206.

Dans un autre exemple, la came peut prendre une forme continue, autrement dit la rainure forme une boucle. Cette boucle peut être de forme sensiblement ellipsoïdale ou sensiblement en étoile. Dans le cas d'une forme en étoile, le sommet de chaque branche est la position la plus éloignée du centre de la roue 204, 206 tandis que le creux séparant chaque branche en est la position la plus proche. Ce genre de configuration en boucle peut s'avérer intéressant lorsqu'il s'agit de réaliser des mouvements cycliques, la fréquence du cycle pouvant être déterminée par le motif de la boucle.

On comprend que la distance radiale de la rainure 242a, 242b par rapport au centre de la roue 204, 206 est variable le long de la rainure 242a, 242b. Autrement dit, la rainure 242a, 242b est non concentrique avec la roue 204, 206.

Deuxième option : rotation ou translation

Dans un autre mode de réalisation, illustré aux figures 7A à 7C, la came 242 décrit un arc concentrique avec la roue 204, 206 et présente une profondeur variable le long de cet arc. En d'autres termes, les première 1a, 1b et seconde 2a, 2b extrémités des cames 242a, 242b sont respectivement situées à équidistance des bords 234, 236 périphériques interne et externe de la roue 204, 206. On comprend donc que la distance radiale par rapport au centre de la roue 204, 206 est constante le long de la rainure 242a, 242b.

Par ailleurs, la profondeur de la came 242a à l'extrémité 1a est supérieure à la profondeur de la came 242a à l'extrémité 2a. Autrement dit, on obtient un gradient de profondeur entre les deux extrémités 1a et 2a de la came 242a. La came 242b est similaire à la came 242a, ainsi la profondeur de la came 242b à l'extrémité 1b est inférieure à la profondeur de la came 242b à l'extrémité 2b. Un gradient de profondeur est donc également obtenu entre les deux extrémités 1b et 2b de la came 242b.

Dans cette configuration, la came 242a, 242b suit une trajectoire circulaire dont la profondeur orientée dans la direction de l'axe X est définie par l'équation :

[Math.4]

$$z = u_0 + v\phi, v = \frac{u_1 - u_0}{2\pi n}, n \in \mathbb{R}[0, 1]$$

Où $z$ représente la position de la mâchoire 208, 210 par rapport à la profondeur de la came 242a, 242b à l'intérieur de l'intervalle $[u_0, u_1]$ appartenant à un nombre réel, $\phi$ représente l'angle et $n$ représente le nombre de tours de la circulaire.

La transmission différentielle telle que décrite dans le mode de réalisation précédent par les équations [Math.2] et [Math.3] s'applique aussi ici, à l'exception que $\eta$ est le pourcentage de translation de la mâchoire 208, 210 dans la plage $[u_0, u_1]$ permis par les plans inclinés des cames 242a, 242b.

En d'autres termes, un déplacement sélectif de la mâchoire 208, 210 est également rendu possible. En effet, une rotation des roues 204, 206 autour de l'axe X dans des sens opposés engendre une translation longitudinale de la mâchoire 208, 210, sans engendrer un déplacement de la mâchoire 208, 210 autour de l'axe X. Comme précédemment, une rotation des roues 204, 206 dans le même sens permet une rotation du manchon 202, sans engendrer de déplacement longitudinal de la mâchoire 208, 210.

Dans ce mode de réalisation, les mâchoires 208, 210 sont positionnées au contact de l'instrument 100 chirurgical et demeurent dans cette position au cours des différentes rotations du fait que les cames 242a, 242b décrivent chacune un arc de cercle concentrique aux roues 204, 206.

Une rotation simultanée d'un quart de tour peut être effectuée pour chaque roue 204, 206, dans un sens opposé ou dans un même sens.

Avec une rotation simultanée d'un quart de tour dans le même sens, les mâchoires 208, 210 sont entraînées en rotation autour de l'axe X. Comme précédemment décrit, le manchon 202 tourne autour de son axe X longitudinal.

On se réfère maintenant aux figures 7B et 7C qui illustrent le positionnement de la mâchoire 208, 210 selon l'axe de coupe T sur la figure 7A.

Sur la figure 7B, le centre de la mâchoire 208, 210 est dans une position initiale et est séparé par une distance W de la face 238 de la roue 204. Les goujons 250, 252 de la mâchoire 208, 210 sont engagés dans la came 242a de la roue 204 au niveau de l'extrémité 2a et dans la came 242b de la roue 206 au niveau de l'extrémité 2b. Durant la rotation simultanée des roues 204, 206, les goujons 250, 252 de la mâchoire 208, 210 suivent le relief, dont l'évolution est inversée, des cames 242a, 242b.

Sur la figure 7C, lorsque la rotation d'un quart de tour est achevée, les goujons 250, 252 se retrouvent à l'extrémité 1a de la came 242a et à l'extrémité 1b de la came 242b. En outre, le centre de la mâchoire 208, 210 s'est déplacé et est séparé de la face 238 par une distance $w$, sensiblement inférieure à la distance W.

Ainsi, avec une rotation simultanée d'un quart de tour dans un sens opposé, les mâchoires 208, 210 se déplacent longitudinalement, selon des axes parallèles à l'axe X longitudinal. Les mâchoires 208, 210 peuvent ainsi se translater sur une distance correspondant à la différence entre la distance W et la distance $w$, et engendrer un mouvement de glissement sur le préhenseur 104.

Il est à noter que la forme de la came présentée ici n'est pas limitative. En effet, la came peut adopter toute forme conduisant à une trajectoire permettant la translation longitudinale ou la rotation d'au moins une mâchoire.

Troisième option : rotation ou pincement avec translation

Dans un autre mode de réalisation, la came 242 décrit un arc non concentrique avec la roue 204, 206 et présente une profondeur variable le long de cet arc. Une première extrémité 1a, 1b de la came 242a, 242b est située à proximité du bord 234 périphérique interne de la roue 204, 206 tandis qu'une seconde extrémité 2a, 2b de la came 242a, 242b est située à proximité du bord 236 périphérique externe, et la profondeur de la came 242a, 242b varie le long de la trajectoire. Autrement dit, ce mode de réalisation est une combinaison des deux premiers modes précédemment décrits et la came 242a, 242b suit une trajectoire telle que définie par les équations [Math.1] et [Math.4]. La came suit donc une trajectoire en spirale en trois dimensions.

Dans ce cas, $\eta$ est en même temps le pourcentage de serrage de la mâchoire 208, 210 dans la plage $[a_0, a_1]$ ainsi que le pourcentage de translation de la mâchoire 208, 210 dans la plage $[u_0, u_1]$ permis par les contraintes physiques imposées par l'intersection des cames 242a, 242b.

En d'autres termes, un déplacement sélectif de la mâchoire 208, 210 est rendu possible. En effet, une rotation des roues 204, 206 autour de l'axe X longitudinal dans des sens opposés engendre simultanément une translation radiale et une translation longitudinale de la mâchoire 208, 210, sans engendrer un déplacement de la mâchoire 208, 210 autour de l'axe X. Une rotation des roues 204, 206 dans le même sens engendre un déplacement de la mâchoire 208, 210 autour de l'axe X longitudinal, et donc du manchon 202 comme explicité précédemment. Par ailleurs, la rotation des roues 204, 206 dans le même sens n'engendre ni déplacement radial ni déplacement longitudinal de la mâchoire 208, 210.

Ce mode de réalisation peut être intéressant dans des cas où le praticien doit actionner une glissière sur le préhenseur 104 et non appuyer dessus pour actionner l'outil 108. En effet, une translation longitudinale de la glissière est nécessaire. Le déplacement simultané de la mâchoire 208, 210 radialement d'une part et longitudinalement d'autre part permet de saisir le préhenseur 104 et de déplacer longitudinalement la mâchoire 208, 210 sur la glissière qui par la suite actionne l'outil chirurgical.

Les options de mouvements précédemment décrites mettent en œuvre un mécanisme 200 muni de deux mâchoires 208, 210. Il est à noter que le nombre de mâchoires 208, 210 n'est pas limitatif. Un résultat identique peut être obtenu avec un mécanisme 200 muni d'une unique mâchoire 208, 210. Dans cette forme de réalisation, la mâchoire 208, 210 comprend un goujon 250, 252 sur chacune de ses faces, de sorte que le premier goujon 250 soit monté mobile dans la première rainure 242a et que le second goujon 252 soit monté mobile dans la seconde rainure 242b.

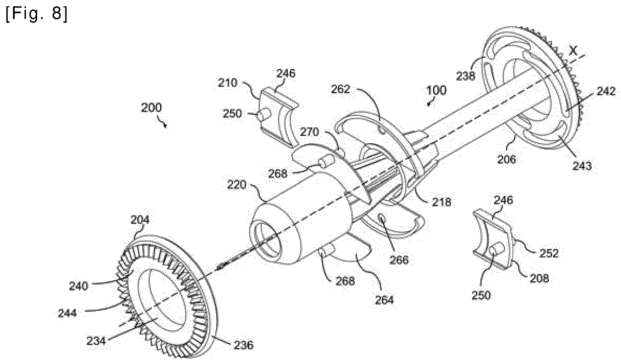

On se réfère à présent à la figure 8 qui représente une autre forme de réalisation du mécanisme 200 d'actionnement.

Dans cette forme de réalisation, le mécanisme d'actionnement 200 est globalement similaire à ce qui a été décrit dans ce qui précède. Il comporte un manchon 202, des roues 204, 206, au moins une mâchoire 208, 210 munie d'un ou plusieurs goujons 250, 252 et des arbres 212, 214 d'entraînement. Les roues 204, 206, l'au moins une mâchoire 208, 210 et les arbres 212, 214 d'entraînement sont similaires et montés comme décrit précédemment.

Le manchon 202 comporte une extrémité 218 amont et une extrémité 220 aval séparables l'une de l'autre. Autrement dit, le manchon 202 n'est pas monobloc. L'extrémité 218 amont comporte au moins un premier guide 262, monté fixe par rapport à l'extrémité 218 amont, par exemple venu de matière avec l'extrémité 218. L'extrémité 220 aval comporte au moins un second guide 264, monté fixe par rapport à l'extrémité 220 aval, par exemple venu de matière avec l'extrémité 220.

L'au moins un premier guide 262 de l'extrémité 218 amont comporte un évidement orienté dans la direction de l'extrémité 220 aval. Au moins un trou 266 traversant est ménagé dans l'au moins un premier guide 262.

L'au moins un second guide 264 de l'extrémité 220 aval comporte un goujon 268 sur une première face et un goujon 270 sur une seconde face, le goujon 270 étant configuré pour passer à travers le trou 266 de l'au moins un premier guide 262. En outre, l'au moins un second guide 264 peut s'engager axialement dans l'évidement de l'au moins un premier guide 262. On comprend que les dimensions du guide 264 sont sensiblement inférieures à celles du guide 262 de sorte que le guide 264 s'engage de manière ajustée dans l'évidement du guide 262.

Le goujon 268 du guide 264 est configuré pour être monté mobile dans une rainure 242 de la roue 204. Le goujon 270 est configuré pour être monté mobile dans une rainure 242 de la roue 206.

En fonctionnement, les roues 204, 206 sont actionnées comme décrit précédemment. Dans l'exemple de la figure 8, chaque roue 204, 206 comporte deux jeux de rainures 242. Dans un premier jeu de rainures 242, identiques et diamétralement opposées, la distance radiale de chaque rainure 242 par rapport au centre de la roue 204, 206 varie le long de la rainure 242. Dans un second jeu de rainures 243, identiques et diamétralement opposées, chaque rainure 243 présente une distance radiale constante le long de la rainure 243 par rapport au centre de la roue 204, 206. Chaque rainure 243 peut avoir un gradient de profondeur le long de la rainure 243.

Pour chaque roue 204, 206, le premier jeu de rainures 242 coopère avec l'au moins une mâchoire 208, 210 par l'intermédiaire des goujons 250, 252 et le second jeu de rainures 243 coopère avec l'au moins un second guide 264 par l'intermédiaire des goujons 268, 270.

Une rotation des roues 204, 206 dans un même sens entraîne, comme précédemment décrit, une rotation du manchon 202 autour de l'axe X longitudinal.

La rotation simultanée des roues 204, 206 dans un des sens opposés entraîne l'actionnement des cames 242, 243 et génère un mouvement simultané des mâchoires 208, 210 et de l'extrémité 220 aval du manchon 202. L'au moins une mâchoire 208, 210 se translate radialement, comme décrit précédemment dans la première option, pour exercer une pression sur le préhenseur 104 de l'instrument 100 chirurgical. L'extrémité 220 aval du manchon 202 se translate longitudinalement, comme décrit précédemment dans la deuxième option. On comprend que cette forme de réalisation est une variante de la troisième option décrite précédemment.

L'intérêt de cette configuration est de pouvoir accommoder un instrument 100 dont la tête 112 se translate lorsqu'une pression s'exerce sur le préhenseur 104. En effet, une translation de la tête 112 nécessite que l'extrémité 220 aval du manchon 202, qui retient vers l'avant l'instrument 100, accompagne le mouvement de translation.

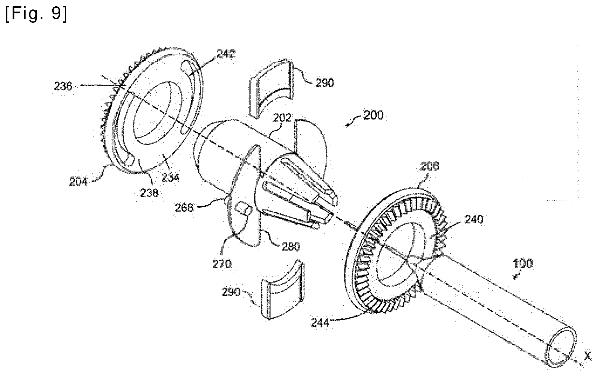

On se réfère maintenant à la figure 9 qui illustre une autre forme de réalisation du mécanisme 200. Dans cette forme de réalisation, une ou plusieurs pièces 280 sont montée(s) fixes par rapport au manchon 202. La ou chaque pièce 280 peut par exemple être venue de matière avec le manchon 202. La ou chaque pièce 280 comporte un ou plusieurs goujons 268, 270, s'étendant selon l'axe X longitudinal du manchon 202, de sorte que l'au moins une rainure 242 de chaque roue 204, 206 reçoive, de façon mobile, un goujon 268, 270.

Dans l'exemple de la figure 9, la pièce 280 est munie d'un goujon 268, 270 sur chacune de ses faces. Le goujon 268 est orienté vers la roue 204 et le goujon 270 vers la roue 206. On comprend donc que le goujon 268 s'engage dans la rainure 242 de la roue 204 et que le goujon 270 s'engage dans la rainure 242 de la roue 206. Au moins une entretoise 290 peut être monté dans le même plan, perpendiculaire à l'axe X longitudinal du manchon 202, que la pièce 280. Cette entretoise 290 maintient un écartement axial fixe entre les roues 204, 206. En outre, l'épaisseur de l'entretoise 290 est sensiblement supérieure à l'épaisseur de la pièce 280.

En fonctionnement, l'instrument 100 chirurgical est introduit dans le manchon 202. Chaque roue 204, 206 comporte au moins une rainure 242 telle que décrite dans la deuxième option précédente, c'est-à-dire avec une profondeur variable le long de la rainure 242 et une position angulaire constante par rapport au centre de la roue 204,206. De la même manière que dans la deuxième option, la rotation des roues 204, 206, en particulier dans des sens opposés, génère une translation longitudinale du manchon 202 entraînée par la pièce 280.

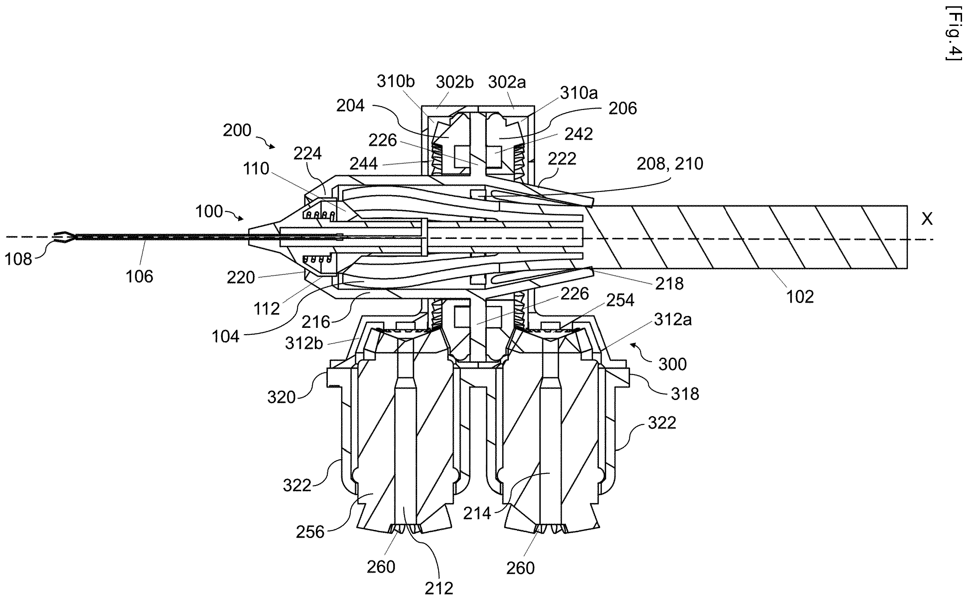

On comprend que la translation longitudinale du manchon 202 permet de translater l'instrument 100 chirurgical. Ceci peut s'avérer particulièrement intéressant dans le cas où l'instrument 100 comprend une seringue et qu'une action de piqûre est nécessaire. Au final, avec les différents modes de réalisation décrits précédemment, plusieurs goujons s'étendant selon l'axe X longitudinal sont prévus sur la ou chaque pièce, sur certaines pièces seulement (voir cas des figures 4, 5 et 9 où les goujons sont soit sur les guides, soit sur les mâchoires), ou encore répartis sur les différentes pièces (voir cas de la figure 8 avec des goujons à la fois sur les guides et les mâchoires).

Le réceptacle 400, la housse de protection, le compartiment de protection 300 et les différents éléments composant le mécanisme 200 d'actionnement énumérés dans ce qui précède peuvent être à usage unique et stériles. Un matériau plastique peut être utilisé. Cela présente le premier avantage de garder un environnement stérile autour du patient durant l'intervention, le préservant d'une quelconque infection. En effet, les éléments étant interchangeables et à usage unique, ils peuvent facilement être montés, notamment par clipsage, sur l'extrémité de l'appareillage au début de l'opération chirurgicale et être jetés une fois celle-ci terminée. Le compartiment 300 comportant le mécanisme 200 peut notamment être changé au cours de la chirurgie si l'utilisation d'un mécanisme 200 particulier comme décrit précédemment est nécessaire. Le second avantage est le faible coût de ces éléments plastiques, ce qui en fait des éléments facilement remplaçables.

En outre, le mécanisme 200 permet à l'instrument 100 chirurgical de se mouvoir avec plusieurs degrés de liberté, en rotation et en translation notamment, mais permet également d'exercer une pression ou un glissement sur un préhenseur 104 de l'instrument 100. Ceci confère l'avantage de pouvoir utiliser une large gamme d'instruments chirurgicaux standards, disponibles sur étagère, et sans nécessiter une modification matérielle de ces derniers.

Enfin, le mécanisme 200 d'actionnement est simple dans sa configuration et n'occupe pas un volume important du fait que l'élément de motorisation, permettant sa mise en œuvre, est déporté dans une partie externe au module 600, partie fixe ou ne nécessitant pas de mouvements complexes. De fait, la compacité accrue du mécanisme 200 permet une diminution de l'encombrement de l'espace de travail, ce qui permet au praticien de garder le patient dans son champ de vision et, en outre, permet l'utilisation d'autres appareillages à proximité de l'invention comme par exemple un microscope utilisé dans le cadre d'une chirurgie vitréo-rétinienne classique ou un autre appareillage semblable à l'invention décrite précédemment.

Il est à noter également que, bien que ne faisant pas partie de l'invention, l'appareillage décrit dans ce qui précède peut être utilisé en combinaison avec une interface de commande et/ou un module logiciel transcrivant les mouvements de l'interface en mouvement du mécanisme d'actionnement. L'interface de commande permet notamment de reproduire avec une grande précision les mouvements du chirurgien, permettant à ce dernier d'agir à distance du patient.

## Revendications

1. Mécanisme d'actionnement (200) pour un instrument (100) chirurgical comportant un manche (102), une gaine (106), un outil (108) et un préhenseur (104) déformable pour actionner l'outil, ledit mécanisme d'actionnement comprenant :

   - un manchon (202), muni d'un axe (X) longitudinal, configuré pour recevoir l'instrument (100) chirurgical et comportant un ou plusieurs guides (226, 264, 280) montés fixes par rapport au manchon et s'étendant sensiblement dans un plan perpendiculaire audit axe longitudinal (X), plusieurs goujons (268, 270) s'étendant selon l'axe longitudinal (X) étant prévus sur le ou chaque guide, sur certains guides seulement, ou encore répartis sur les différents guides, ou une ou plusieurs mâchoires (208, 210) montées entre des guides (226, 264, 280) montés fixes par rapport au manchon, de sorte à pouvoir translater par rapport auxdits guides et s'étendant sensiblement dans le plan perpendiculaire audit axe longitudinal (X), plusieurs goujons (250, 252) s'étendant selon l'axe longitudinal (X) étant prévus sur la ou chaque mâchoire, sur certaines mâchoires seulement, ou encore répartis sur les différentes mâchoires ;
   - au moins deux roues (204, 206), montées sur le manchon (202) de part et d'autre du ou desdits guides ou de la ou desdites mâchoires selon ledit axe longitudinal (X), chaque roue (204, 206) étant munie d'au moins une rainure (242, 242a, 242b, 243) et d'un élément (244) de transmission mécanique, ladite au moins une rainure (242, 242a, 242b, 243) de chaque roue (204, 206) recevant, de façon mobile dans ladite rainure, l'un de ladite pluralité de goujons (250, 252, 268, 270) ;
   - au moins deux arbres d'entraînement (212, 214), un premier arbre d'entraînement (212) étant muni d'un premier élément (254) de transmission mécanique coopérant avec l'élément (244) de transmission mécanique de l'une (204) des deux roues (204, 206) et un deuxième arbre d'entraînement (214) étant muni d'un autre premier élément (254) de transmission mécanique coopérant avec l'élément (244) de transmission mécanique de l'autre (206) des deux roues (204, 206).

2. Mécanisme d'actionnement (200) selon la revendication 1, dans lequel le manchon (202) comporte une butée (224) interne s'étendant radialement au niveau d'une première extrémité (220) dudit manchon, et des languettes flexibles (222) s'étendant axialement au niveau d'une seconde extrémité (218).

3. Mécanisme d'actionnement (200) selon la revendication 1 ou 2, dans lequel l'élément (244, 254, 260) de transmission mécanique est un engrenage ou une poulie entraînée par courroie.

4. Mécanisme d'actionnement (200) selon l'une des revendications précédentes, dans lequel la rainure (242, 242a, 242b, 243) présente une distance radiale par rapport au centre de la roue (204, 206) qui varie le long de ladite rainure.

**5.** Mécanisme d'actionnement (200) selon l'une des revendications 1 à 3, dans lequel la rainure (242, 242a, 242b, 243) présente une distance radiale par rapport au centre de la roue (204, 206), laquelle est constante le long de ladite rainure.

**6.** Mécanisme d'actionnement (200) selon l'une des revendications 1 à 5, dans lequel la rainure (242, 242a, 242b, 243) présente une profondeur variable le long de ladite rainure.

**7.** Mécanisme d'actionnement (200) selon l'une des revendications 1 à 3, dans lequel chaque roue (204, 206) comprend au moins une première rainure (242) présentant une distance radiale par rapport au centre de la roue (204, 206) qui varie le long de ladite première rainure et présentant une profondeur variable le long de ladite première rainure, et au moins une seconde rainure (243), différente de ladite première rainure (242) présentant une distance radiale par rapport au centre de la roue (204, 206) qui est constante le long de ladite seconde rainure et présentant une profondeur variable le long de ladite seconde rainure.

**8.** Module (600) **caractérisé en ce qu'**il comprend un mécanisme (200) d'actionnement tel que décrit à l'une des revendications précédentes, ledit mécanisme (200) étant logé dans un compartiment (300) de protection, maintenant en interaction les éléments dudit mécanisme (200) d'actionnement.

**9.** Module (600) selon la revendication précédente, comprenant un réceptacle (400) amovible configuré pour recevoir le compartiment (300) de protection, ledit réceptacle (400) étant fixé à une extrémité (500) d'un appareillage.

**10.** Module (600) selon la revendication précédente, dans lequel au moins deux arbres (502a, 502b) parallèles d'entraînement, reliés mécaniquement à un élément de motorisation, se trouvent en saillie de ladite extrémité (500), chaque arbre (502a, 502b) étant configuré pour coopérer avec un second élément (260) de transmission mécanique d'un desdits au moins deux arbres d'entraînement (212, 214) du mécanisme d'actionnement (200).

**11.** Module (600) selon l'une des revendications 8 à 10, dans laquelle une housse de protection est intégrée au réceptacle (400) de sorte que ladite housse entoure une partie opposée au module (600).

**12.** Module (600) selon la revendication précédente, dans lequel le réceptacle (400), le compartiment (300), le mécanisme (200) et la housse sont des équipements stériles à usage unique.

**13.** Procédé de mise en œuvre d'un mécanisme d'actionnement (200) selon l'une des revendications précédentes, ledit procédé étant réalisé en dehors du patient, dans lequel une rotation dans le même sens des roues (204, 206) engendre une rotation du manchon (202) autour de son axe longitudinal.

**14.** Procédé de mise en œuvre d'un mécanisme d'actionnement (200) selon le procédé de la revendication 13 et selon le mécanisme de la revendication 4, ledit procédé étant réalisé en dehors du patient, dans lequel une rotation dans des sens opposés des roues (204, 206) engendre une translation radiale d'au moins une mâchoire (208, 210) de sorte que ladite mâchoire (208, 210) exerce une pression sur l'instrument (100) chirurgical.

**15.** Procédé de mise en œuvre d'un mécanisme d'actionnement (200) selon le procédé de la revendication 13 et selon le mécanisme de la combinaison des revendications 5 et 6, ledit procédé étant réalisé en dehors du patient, dans lequel une rotation dans des sens opposés des roues (204, 206) engendre une translation longitudinale d'au moins une mâchoire (208, 210) ou du manchon (202) par l'intermédiaire d'un guide (264, 280) le long de l'instrument (100) chirurgical.

**16.** Procédé de mise en œuvre d'un mécanisme d'actionnement (200) selon le procédé de la revendication 13 et selon le mécanisme de la combinaison des revendications 4 et 6, ledit procédé étant réalisé en dehors du patient, dans lequel une rotation des roues (204, 206) dans des sens opposés engendre simultanément une translation longitudinale et une translation radiale d'au moins une mâchoire (208, 210) de sorte que ladite mâchoire (208, 210) exerce une pression sur l'instrument (100) chirurgical tout en se déplaçant longitudinalement le long dudit instrument (100).

**17.** Procédé de mise en œuvre d'un mécanisme d'actionnement (200) selon le procédé de la revendication 13 et selon le mécanisme de la revendication 7, ledit procédé étant réalisé en dehors du patient, dans lequel une rotation des roues (204, 206) dans des sens opposés engendre simultanément une translation longitudinale du manchon (202) par l'intermédiaire d'un guide (264, 280) le long de l'instrument chirurgical et une translation radiale d'au moins une mâchoire (208, 210) de sorte que ladite mâchoire exerce une pression sur l'instrument (100) chirurgical.

**Patentansprüche**

1. Betätigungsmechanismus (200) für ein chirurgisches Instrument (100), umfassend einen Griff (102), eine Hülse (106), ein Werkzeug (108) und einen verformbaren Greifer (104) zum Betätigen des Werkzeugs, wobei der Betätigungsmechanismus umfasst:

   - eine mit einer Längsachse (X) versehene Muffe (202), die konfiguriert ist, um das chirurgische Instrument (100) aufzunehmen und eine oder mehrere Führungen (226, 264, 280), die in Bezug auf die Muffe fest montiert sind und sich im Wesentlichen in einer Ebene senkrecht zur Längsachse (X) erstrecken, mehrere Bolzen (268, 270), die sich gemäß der Längsachse (X) erstrecken, auf der oder jeder Führung, nur auf gewissen Führungen oder auf den unterschiedlichen Führungen verteilt vorgesehen sind, oder eine oder mehrere Klemmbacken (208, 210), die zwischen den in Bezug auf die Muffe fest montierten Führungen (226, 264, 280) derart montiert sind, um sich in Bezug auf die Führungen verschieben zu können und sich im Wesentlichen in der Ebene senkrecht zur Längsachse (X) zu erstrecken, umfasst, wobei mehrere Bolzen (250, 252), die sich gemäß der Längsachse (X) erstrecken, auf der oder jeder Klemmbacke, nur auf gewissen Klemmbacken oder auf den unterschiedlichen Klemmbacken verteilt vorgesehen sind;
   - mindestens zwei an der Muffe (202), beiderseits der Führung oder der Führungen oder der Klemmbacke oder den Klemmbacken gemäß der Längsachse (X) montierte Räder (204, 206), wobei jedes Rad (204, 206) mit mindestens einer Rille (242, 242a, 242b, 243) und einem Element (244) zur mechanischen Übertragung versehen ist, wobei die mindestens eine Rille (242, 242a, 242b, 243) von jedem Rad (204, 206) einen der Vielzahl von Bolzen (250, 252, 268, 270) auf bewegliche Weise aufnimmt;
   - mindestens zwei Antriebswellen (212, 214), wobei eine erste Antriebswelle (212) mit einem ersten Element (254) zur mechanischen Übertragung, das mit dem Element (244) zur mechanischen Übertragung von einem (204) der zwei Räder (204, 206) zusammenwirkt, versehen ist, und eine zweite Antriebswelle (214), die mit einem anderen ersten Element (254) zur mechanischen Übertragung von dem anderen (206) der zwei Räder (204, 206) zusammenwirkt, versehen ist.

2. Betätigungsmechanismus (200) nach Anspruch 1, wobei die Muffe (202) einen internen Anschlag (224), der sich auf Höhe eines ersten Endes (220) der Muffe radial erstreckt, und flexible Laschen (222) umfasst, die sich auf Höhe eines zweiten Endes (218) axial erstrecken.

3. Betätigungsmechanismus (200) nach Anspruch 1 oder 2, wobei das Element (244, 254, 260) zur mechanischen Übertragung eine Verzahnung oder eine durch einen Riemen angetriebene Riemenscheibe ist.

4. Betätigungsmechanismus (200) nach einem der vorstehenden Ansprüche, wobei die Rille (242, 242a, 242b, 243) in Bezug auf die Mitte des Rads (204, 206) einen radialen Abstand aufweist, der entlang der Rille variiert.

5. Betätigungsmechanismus (200) nach einem der Ansprüche 1 bis 3, wobei die Rille (242, 242a, 242b, 243) in Bezug auf die Mitte des Rads (204, 206) einen radialen Abstand aufweist, der entlang der Rille konstant ist.

6. Betätigungsmechanismus (200) nach einem der Ansprüche 1 bis 5, wobei die Rille (242, 242a, 242b, 243) entlang der Rille eine variable Tiefe aufweist.

7. Betätigungsmechanismus (200) nach einem der Ansprüche 1 bis 3, wobei jedes Rad (204, 206) mindestens eine erste Rille (242), die einen radialen Abstand in Bezug auf die Mitte des Rads (204, 206) aufweist, der entlang der ersten Rille variiert, und die entlang der ersten Rille eine variable Tiefe aufweist, und mindestens eine zweite Rille (243) umfasst, die sich von der ersten Rille (242) unterscheidet, die einen in Bezug auf die Mitte des Rads (204, 206) radialen Abstand aufweist, der entlang der zweiten Rille konstant ist, und die eine entlang der zweiten Rille variable Tiefe aufweist.

8. Modul (600), **dadurch gekennzeichnet, dass** es einen Betätigungsmechanismus (200) wie in einem der vorstehenden Ansprüche beschrieben umfasst, wobei der Mechanismus (200) in einem Schutzkompartiment (300) untergebracht ist, das die Elemente des Betätigungsmechanismus (200) in Interaktion hält.

9. Modul (600) nach dem vorstehenden Anspruch, umfassend einen abnehmbaren Behälter (400), der zum Aufnehmen des Schutzkompartiments (300) konfiguriert ist, wobei der Behälter (400) an einem Ende (500) eines Geräts befestigt ist.

10. Modul (600) nach dem vorstehenden Anspruch, wobei sich mindestens zwei parallele Antriebswellen (502a, 502b), die mechanisch mit einem Motorelement verbunden sind, von dem Ende (500) hervorstehend befinden, wobei jede Welle (502a, 502b) konfiguriert ist, um mit einem zweiten Element (260) zur mechanischen Übertragung von einer der mindestens zwei Antriebswellen (212, 214) des Betätigungsmechanismus (200) zusammenzuwirken.

11. Modul (600) nach einem der Ansprüche 8 bis 10, wobei eine Schutzhülle mit dem Behälter (400) derart integriert ist, dass die Hülle einen dem Modul (600) gegenüberliegenden Teil umgibt.

12. Modul (600) nach dem vorstehenden Anspruch, wobei der Behälter (400), das Kompartiment (300), der Mechanismus (200) und die Hülle sterile Einrichtungen zur einmaligen Verwendung sind.

13. Verfahren zur Implementierung eines Betätigungsmechanismus (200) nach einem der vorstehenden Ansprüche, wobei das Verfahren außerhalb des Patienten implementiert wird, wobei eine Drehung der Räder (204, 206) in der gleichen Richtung eine Drehung der Muffe (202) um ihre Längsachse verursacht.

14. Verfahren zur Implementierung eines Betätigungsmechanismus (200) nach dem Verfahren von Anspruch 13 und nach dem Mechanismus von Anspruch 4, wobei das Verfahren außerhalb des Patienten implementiert wird, wobei eine Drehung der Räder (204, 206) in entgegengesetzte Richtungen eine radiale Verschiebung von mindestens einer Klemmbacke (208, 210) derart verursacht, dass die Klemmbacke (208, 210) einen Druck auf das chirurgische Instrument (100) ausübt.

15. Verfahren zur Implementierung eines Betätigungsmechanismus (200) nach dem Verfahren von Anspruch 13 und nach dem Mechanismus der Kombination von Ansprüchen 5 und 6, wobei das Verfahren außerhalb des Patienten implementiert wird, wobei eine Drehung der Räder (204, 206) in entgegengesetzte Richtungen eine Längsverschiebung von mindestens einer Klemmbacke (208, 210) oder der Muffe (202) mittels einer Führung (264, 280) entlang des chirurgischen Instruments (100) verursacht.

16. Verfahren zur Implementierung eines Betätigungsmechanismus (200) nach dem Verfahren von Anspruch 13 und nach dem Mechanismus der Kombination von Ansprüchen 4 und 6, wobei das Verfahren außerhalb des Patienten implementiert wird, wobei eine Drehung der Räder (204, 206) in entgegengesetzte Richtungen gleichzeitig eine Längsverschiebung und eine radiale Verschiebung von mindestens einer Klemmbacke (208, 210) derart verursacht, dass die Klemmbacke (208, 210) einen Druck auf das chirurgische Instrument (100) ausübt, indem sie sich in Längsrichtung entlang des Instruments (100) bewegt.

17. Verfahren zur Implementierung eines Betätigungsmechanismus (200) nach dem Verfahren von Anspruch 13 und nach dem Mechanismus von Anspruch 7, wobei das Verfahren außerhalb des Patienten implementiert wird, wobei eine Drehung der Räder (204, 206) in entgegengesetzte Richtungen gleichzeitig eine Längsverschiebung der Muffe (202) mittels einer Führung (264, 280) entlang des chirurgischen Instruments und eine radiale Verschiebung von mindestens einer Klemmbacke (208, 210) derart verursacht, dass die Klemmbacke einen Druck auf das chirurgische Instrument (100) ausübt.

**Claims**

1. An actuation mechanism (200) for a surgical instrument (100) comprising a handle (102), a sheath (106), a tool (108) and a deformable gripper (104) for actuating the tool, said actuation mechanism comprising:

   - a sleeve (202), equipped with a longitudinal axis (X), configured to receive the surgical instrument (100) and comprising one or more guides (226, 264, 280) stationary mounted with respect to the sleeve and extending substantially in a plane perpendicular to said longitudinal axis (X), a plurality of studs (268, 270) extending along the longitudinal axis (X) being provided on the or each guide, on some guides only, or distributed over the different guides, or a plurality of jaws (208, 210) mounted between the guides (226, 264, 280) stationary mounted with respect to the sleeve, so as to be translate relative to said guides and extending substantially in the plane perpendicular to said longitudinal axis (X), several studs (250, 252) extending along the longitudinal axis (X) being provided on the or each jaw, on some jaws only, or distributed on the different jaws ;
   - at least two wheels (204, 206), mounted on the sleeve (202) on either side of said part or parts along said longitudinal axis (X), each wheel (204, 206) being equipped with at least one groove (242, 242a, 242b, 243) and a mechanical transmission element (244), said at least one groove (242, 242a, 242b, 243) of each wheel (204, 206) receiving, in a movable manner in said groove, one of said plurality of studs (250, 252, 268, 270);
- at least two drive shafts (212, 214), a first drive shaft (212) being equipped with a first mechanical transmission element (254) cooperating with the mechanical transmission element (244) of one (204) of the two wheels (204, 206) and a second drive shaft (214) being equipped with another first mechanical transmission element (254) cooperating with the mechanical transmission element (244) of the other (206) of the two wheels (204, 206).

2. The actuation mechanism (200) of claim 1, wherein the sleeve (202) comprises an internal abutment (224) extending radially at the level of a first end (220) of said sleeve, and flexible tongues (222) extending axially at the level of a second end (218).

3. The actuation mechanism (200) according to claim 1 or 2, wherein the mechanical transmission element (244, 254, 260) is a gear or a belt driven pulley.

4. The actuation mechanism (200) according to any of the preceding claims, wherein the groove (242, 242a, 242b, 243) has a radial distance with respect to the centre of the wheel (204, 206) that varies along said groove.

5. The actuation mechanism (200) according to any of claims 1 to 3, wherein the groove (242, 242a, 242b, 243) has a radial distance with respect to the centre of the wheel (204, 206), which is constant along said groove.

6. The actuation mechanism (200) according to any of claims 1 to 5, wherein the groove (242, 242a, 242b, 243) has a variable depth along said groove.

7. The actuation mechanism (200) according to any one of claims 1 to 3, wherein each wheel (204, 206) comprises at least one first groove (242) having a radial distance with respect to the centre of the wheel (204, 206) which varies along said first groove and having a variable depth along said first groove, and at least one second groove (243), different from said first groove (242) having a radial distance with respect to the centre of the wheel (204, 206) which is constant along said second groove and having a variable depth along said second groove.

8. A module (600) **characterised in that** it comprises an actuation mechanism (200) as described in one of the preceding claims, said mechanism (200) being housed in a protective compartment (300), maintaining the elements of said actuation mechanism (200) in interaction.

9. The module (600) according to the preceding claim, comprising a removable receptacle (400) configured to receive the protective compartment (300), said receptacle (400) being attached to one end (500) of an apparatus.

10. The module (600) according to the preceding claim, wherein at least two parallel drive shafts (502a, 502b), mechanically connected to a motorisation element, are located projecting from said end (500), each shaft (502a, 502b) being configured to cooperate with a second mechanical transmission element (260) of one of said at least two drive shafts (212, 214) of the actuation mechanism (200).

11. The module (600) according to any one of claims 8 to 10, wherein a protective cover is integrated into the receptacle (400) such that said cover surrounds a portion opposite the module (600).

12. The module (600) according to the preceding claim, wherein the receptacle (400), the compartment (300), the mechanism (200) and the cover are sterile single-use equipment.

13. A method for implementing an actuation mechanism (200) according to any of the preceding claims, said method being carried out outside the patient, wherein a rotation in the same direction of the wheels (204, 206) causes a rotation of the sleeve (202) about its longitudinal axis.

14. A method for implementing an actuation mechanism (200) according to the method of claim 13 and according to the mechanism of claim 4, said method being carried out outside the patient, wherein a rotation in opposite directions of the wheels (204, 206) causes a radial translation of at least one part (208, 210) so that said part (208, 210) exerts a pressure on the surgical instrument (100).

15. A method for implementing an actuation mechanism (200) according to the method of claim 13 and according to the mechanism of the combination of claims 5 and 6, said method being carried out outside the patient, wherein a rotation in opposite directions of the wheels (204, 206) causes a longitudinal translation of at least one part (208, 210, 264, 280) along the surgical instrument (100).

16. A method for implementing an actuation mechanism (200) according to the method of claim 13 and according to the mechanism of the combination of claims 4 and 6, said method being carried out outside the patient, wherein a rotation of the wheels (204, 206) in opposite directions simultaneously causes a longitudinal translation and a radial translation of at least one part (208, 210) so that said part (208, 210) exerts a pressure on the surgical instrument (100) while displacing longitudinally along said instrument (100).

17. A method for implementing an actuation mechanism (200) according to the method of claim 13 and according to the mechanism of claim 7, said method being carried out outside the patient, wherein a rotation of the wheels (204, 206) in opposite directions simultaneously causes a longitudinal translation of at least one part (264, 280) along the surgical instrument and a radial translation of at least one other part (208, 210) such that said other part exerts a pressure on the surgical instrument (100).

[Fig.1]

[Fig.2]

[Fig.3]

[Fig.4]

[Fig.5]

[Fig.6A]

[Fig.6B]

[Fig.6C]

[Fig.7A]

[Fig.7B]

[Fig.7C]

[Fig. 8]

[Fig. 9]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2019183236 A1